# EUROPEAN PATENT SPECIFICATION

(11) **EP 3 851 029 B1**
(45) Date of publication and mention of the grant of the patent: **27.05.2026**
(21) Application number: 19877903.5
(22) Date of filing: 01.11.2019
(51) Int. Cl.: A61B 5/00, G01V 8/10, A61B 5/024

(54) **HEART RATE DETECTION METHOD AND ELECTRONIC DEVICE**
VERFAHREN ZUR HERZFREQUENZDETEKTION UND ELEKTRONISCHE VORRICHTUNG
PROCÉDÉ DE DÉTECTION DE FRÉQUENCE CARDIAQUE ET DISPOSITIF ÉLECTRONIQUE

(30) Priority: 01.11.2018 CN 201811294545; 14.02.2019 CN 201910114923
(43) Date of publication of application: 21.07.2021
(73) Proprietor: HUAWEI TECHNOLOGIES CO., LTD., Shenzhen, Guangdong 518129 (CN)
(72) Inventor: XI, Yi, Shenzhen, Guangdong 518129 (CN); SUN, Shiyou, Shenzhen, Guangdong 518129 (CN); HE, Yanguo, Shenzhen, Guangdong 518129 (CN)
(74) Representative: Thun, Clemens
(86) International application number: PCT/CN2019/114974
(87) International publication number: WO 2020/088639

(56) References cited:
- WO-A1-2009/088799
- CN-A- 107 820 410
- CN-A- 107 907 916
- CN-A- 108 337 903
- CN-A- 108 564 179
- US-A1- 2016 027 282
- US-A1- 2017 215 747
- US-A1- 2018 000 363

## Description

### TECHNICAL FIELD

Embodiments of this application relate to the field of electronic technologies, and in particular, to a method and an electronic device for detecting a heart rate.

### BACKGROUND

Exercise and health detection such as exercise intensity detection, precise sleep detection, precise calorie detection, or sleep apnea recognition is performed by using a wrist strap. The exercise and health detection usually needs to be performed based on heart rate measurement. A technology such as photoplethysmograph (photoplethysmograph, PPG) may be used to measure a heart rate.

Because of a physical principle of the PPG, a PPG element needs to be attached to the skin to obtain a good signal, to obtain an accurate heart rate through measurement. Once there is a relatively large gap between the PPG element and the skin, a PPG signal is lost, and consequently heart rate measurement is inaccurate.

The document US 2017/ 215747 A1 shows an optical vital signs sensor worn on the skin of a user, using two different wave length of light to detect if the sensor is correctly worn, and to detect a heart rate.

The document WO 2009/ 088799 A1 shows a method and apparatus for assessing contact of a sensor with arterialized tissue. Especially, the use of light of two wave lengths to detect if the sensor is in the correct position is shown.

The document US 2018/000363 A1 shows a PPG sensor for performing measurements on human skin.

### SUMMARY

The present invention is defined by the independent claims. Further advantageous developments are shown by the dependent claims.

The embodiments of this application provide a method and an electronic device for detecting a heart rate, to detect the heart rate more accurately.

### BRIEF DESCRIPTION OF DRAWINGS

FIG. 1 is a schematic structural diagram of an electronic device according to an embodiment of this application;
FIG. 2 is a schematic structural diagram of a wrist strap according to an embodiment of this application;
FIG. 3 is a schematic structural diagram of a smartwatch according to an embodiment of this application;
FIG. 4 is a schematic structural diagram of another smartwatch according to an embodiment of this application;
FIG. 5 is a schematic structural diagram of another smartwatch according to an embodiment of this application;
FIG. 6 is a schematic structural diagram of another smartwatch according to an embodiment of this application;
FIG. 7 is a schematic structural diagram of another smartwatch according to an embodiment of this application;
FIG. 8 is a schematic structural diagram of another smartwatch according to an embodiment of this application;
FIG. 9 is a schematic structural diagram of another smartwatch according to an embodiment of this application;
FIG. 10 is a schematic flowchart of a method for detecting a heart rate according to an embodiment of the present invention;
FIG. 11 is an example of an electronic device according to an embodiment of the present invention; and
FIG. 12 is a schematic structural diagram of an apparatus for detecting a heart rate according to an embodiment of the present invention.

### DESCRIPTION OF EMBODIMENTS

The following describes the embodiments of this application with reference to the accompanying drawings in the embodiments of this application.

A wearing status may be determined by using an optical signal of a light color. However, a signal is affected by many interference factors, and the interference factors are also variable. For example, differences in external ambient light intensity, external ambient temperature, human skin color depth, human skin roughness, human skin moisture content, human skin fat content, and the like can all affect the optical signal, which finally affects a determining result, so that a misjudgment rate of determining the wearing status is relatively high. This finally affects accuracy of heart rate detection and user experience.

Based on the foregoing problem, the embodiments of the present invention provide a method for detecting a heart rate and an electronic device. Based on a principle that anti-interference capabilities between optical signals of different
interference factors and different colors are different, in the embodiments of the present invention, the wearing status of a wrist strap is detected based on colored lights of at least two colors. This improves accuracy of wrist strap status detection and improves user experience.

The embodiments of the present invention are applicable to an electronic device. The electronic device may be a wrist strap, a handheld electronic device that can communicate with the wrist strap, or the like. The wrist strap may include one or more optical transmitters and one or more optical sensors. Sending a light by using the one or more optical transmitters and receiving a light by using the one or more optical sensors may determine lights of at least two colors on a preset optical path. The lights of the at least two colors may be used to measure the wearing status of a user. A part of the light from the optical transmitter may be absorbed by skin, a vasculature, blood, or the like, and a part of the light may be reflected back to the optical sensor that is located at a same position as the optical transmitter.

In some examples, the device may further include another component, for example, one or more lenses and/or reflectors, to increase signal strength and/or shield the optical transmitter, the optical sensor, and associated wiring from being seen by the user.

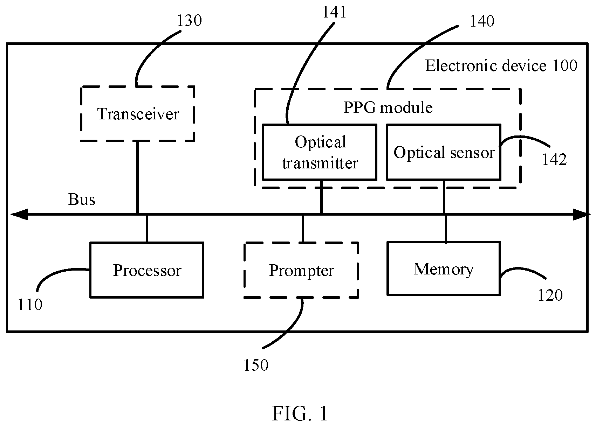

FIG. 1 is a schematic structural diagram of an electronic device according to an embodiment of this application. As shown in FIG. 1, the electronic device 100 includes a processor 110, a memory 120, an optical transmitter 141, and an optical sensor 142. The processor 110, the memory 120, the optical transmitter 141, and the optical sensor 142 may be connected by using a bus (not shown in the figure). There may be one or more optical transmitters 141 and one or more optical sensors 142. The optical transmitter 141 is configured to send lights of at least two colors on a preset optical path, and each optical transmitter 141 sends lights of one or more colors on the preset optical path. The optical sensor 142 is configured to detect the preset optical path. The optical sensor 142 is coupled to the processor 110, to send detected lights to the processor 110. For example, the optical sensor 142 is connected to the processor 110 by using the bus. The memory 120 is configured to store a program and data. The processor 110 is configured to execute the program stored in the memory 120, read the data stored in the memory 120, and determine a wearing status of the electronic device based on the lights that are of at least two colors and that are detected by the optical sensor 142. Further, the processor 110 may detect a heart rate with reference to the wearing status of the electronic device.

There may be one or more preset optical paths, and each preset optical path is formed by one optical transmitter and one optical sensor. The plurality of preset optical paths may include optical transmitters and/or optical sensors in different positions. In some examples, the plurality of preset optical paths may include different path positions. In some examples, the plurality of preset optical paths may include overlapping and collinear paths (namely, along a same line).

In an optional embodiment, the electronic device 100 may further include a transceiver 130. The transceiver is configured to communicate with another electronic device. The another electronic device includes a wrist strap or a mobile phone. For example, the electronic device 100 may send a determined wearing status to the another electronic device by using the transceiver 130.

In another optional embodiment, the electronic device may further include a PPG module 1140, and the PPG module includes the optical transmitter 141 and an optical sensor 142.

In the claimed invention, the electronic device includes a prompter 150. The prompter 150 is connected to the processor 110, and is configured to generate prompt information based on an instruction of the processor. The prompt information is used to prompt the wearing status of the electronic device. For example, the prompter 150 may be a display, and the prompt information may prompt a user of the wearing status of the electronic device in a form of an image and a text. For another example, the prompter 150 may alternatively be a speaker, and the prompt information may prompt the user of the wearing status of the electronic device in a form of audio. For another example, the prompter 150 may be a buzzer, and the prompt information may prompt the user of the wearing status of the electronic device in a form of vibration.

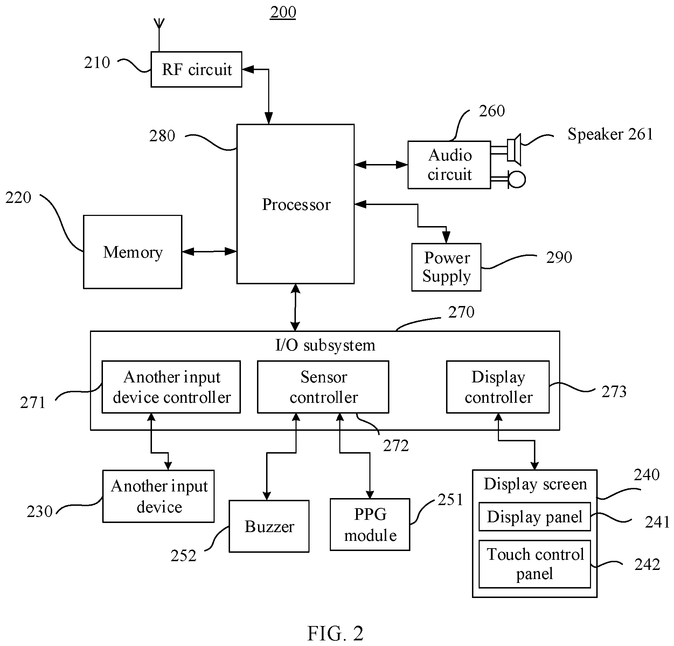

The following further describes this application by using a wrist strap 200 as an example of the electronic device 100. FIG. 2 is a schematic structural diagram of a wrist strap according to an embodiment of the present invention. As shown in FIG. 2, the wrist strap 200 includes components such as an RF (Radio Frequency, radio frequency) circuit 210, a memory 220, another input device 230, a touchscreen 240, a PPG module 251, a buzzer 252, an audio circuit 260, an I/O subsystem 270, a processor 280, and a power supply 290. A person skilled in the art may understand that the structure of the mobile phone shown in FIG. 2 does not constitute a limitation on the wrist strap, and the wrist strap may include more or fewer components than those shown in the figure, or some components may be combined, or some components may be split, or a component may be deployed differently.

The following describes in detail the components of the wrist strap 200 with reference to FIG. 1.

The RF circuit 210 may be configured to receive and send signals in an information sending and receiving process or a call process. In particular, after receiving downlink information from a base station, the RF circuit 210 sends the downlink information to the processor 280 for processing. In addition, the RF circuit 210 sends uplink data to the base station. The RF circuit usually includes but is not limited to an antenna, at least one amplifier, a transceiver, a coupler, an LNA (Low Noise Amplifier, low noise amplifier), a duplexer, and the like. In addition, the RF circuit 210 may further communicate with a network and another device through wireless communication. The wireless communication may use any communications standard or protocol, including but not limited to global system for mobile communications (global system of mobile communication, GSM), a general packet radio service (general packet radio service, GPRS), code division multiple access (code division multiple access, CDMA), wideband code division multiple access (wideband code division multiple access, WCDMA), long term evolution (long term evolution, LTE), an email, a short message service (short message service, SMS), and the like.

The memory 220 may be configured to store a software program. The processor 280 executes various functions of the wrist strap 200 by running the software program stored in the memory 220. The memory 220 may mainly include a program storage area and a data storage area. The program storage area may store an operating system, an application program required by at least one function (for example, a sound playing function or an image playing function), and the like. The data storage area may store data (for example, audio data or an address book) maintained based on use of the wrist strap 2100, and the like. In addition, the memory 220 may include a high speed random access memory, and may further include a nonvolatile memory, such as at least one magnetic disk storage device, a flash memory, or another volatile solid-state storage device.

The another input device 230 may be configured to receive input digit or character information, and generate a key signal input related to user setting and function control of the wrist strap 200. Specifically, the another input device 230 may include but is not limited to: one or more of a physical keyboard, a function key (such as a volume control key or an on/off key), a trackball, a mouse, a joystick, an optical mouse (the optical mouse is a touch-sensitive surface that does not display visual output, or an extension of a touch-sensitive surface formed by a touchscreen), and the like. The another input device 230 is connected to another input device controller 271 of the I/O subsystem 270, and exchanges a signal with the processor 280 under control of the another input device controller 271.

The touchscreen 240 may be configured to display information entered by a user or information provided to the user, and various menus of the wrist strap 200, and may further receive a user input. Specifically, the touchscreen 240 may include a display panel 241 and a touch panel 242. The display panel 241 may be configured in a form of a liquid crystal display (liquid crystal display, LCD), an organic light-emitting diode (organic light-emitting diode, OLED), or the like. The touch panel 242 is also referred to as a touchscreen, a touch-sensitive screen, or the like, and may collect a contact or noncontact operation of the user on or near the touch panel 242 (such as an operation of the user on or near the touch panel 242 by using any suitable object or attachment such as a finger or a stylus, or a motion sensing operation, where the operation includes a single-point control operation, a multi-point control operation, and another type of operation), and drive a corresponding connection apparatus based on a preset program. Optionally, the touch panel 242 may include two parts: a touch detection apparatus and a touch controller. The touch detection apparatus detects a touch orientation and gesture of the user, detects a signal brought by the touch operation, and sends the signal to the touch controller. The touch controller receives touch information from the touch detection apparatus, converts the touch information into information that can be processed by the processor, then sends the information to the processor 280, and can receive and execute a command sent by the processor 280. In addition, the touch panel 242 may be implemented by using a plurality of types such as a resistive type, a capacitive type, an infrared ray, and a surface acoustic wave, or the touch panel 242 may be implemented by using any technology developed in the future. Further, the touch panel 242 may cover the display panel 241. The user may perform, based on content (the displayed content includes, but is not limited to, a soft keyboard, a virtual mouse, a virtual key, an icon, and the like) displayed on the display panel 241, an operation on or near the touch panel 242 covering the display panel 241. After detecting the operation on or near the touch panel 242, the touch panel 242 transfers the operation to the processor 280 by using the I/O subsystem 270, to determine the user input. Subsequently, the processor 280 provides a corresponding visual output on the display panel 241 by using the I/O subsystem 270 based on the user input. In FIG. 1, the touch panel 242 and the display panel 241 are used as two independent parts to implement input and output functions of the wrist strap 200. However, in some embodiments, the touch panel 242 and the display panel 241 can be integrated to implement the input and output functions of the wrist strap 200.

The display panel 241 may provide, under control of a program of the processor 280, prompt information such as a wearing manner and a wearing status, and historical information in a visual (digital, table, or graph) or a listenable (synthetic speech or tone) form of a detected heart rate. As a nonlimiting example, a visual curve diagram may be displayed. The visual curve diagram shows a heart rate calculated every 5 minutes during a previous fixed time interval (for example, 1 hour) or after an exercise period has ended (as determined by an indication from the user). The display panel 241 may further provide, under control of the processor 280, average heart rate information or heart rate statistics in one or more previous time periods. In another example, a current heart rate value may be provided on the display panel 241 as a "real-time" heart rate value periodically (for example, every second) displayed to the user during a process of an ongoing exercise plan.

The PPG module 251 includes an optical transmitter and an optical sensor. Measuring a heart rate by using the PPG module is based on a principle of absorption of a light by a substance. The optical transmitter in the PPG module of the electronic device irradiates a blood vessel of skin, and the optical sensor receives a light that is transmitted from the skin. Because different volumes of blood in the blood vessel absorb green light differently, when the heart beats, a blood flow increases, and absorption of the green light will increase accordingly. The blood flow is reduced when in a heartbeat gap, and the absorption of the green light is also reduced. Therefore, the heart rate can be measured based on absorbance of the blood. In an operation, the optical transmitter may transmit a light beam to skin of the user, and the light beam may be reflected by the skin of the user and received by the optical sensor. The optical sensor may convert the light into an electrical signal indicating intensity of the light. The electrical signal may be in an analog form, and may be converted into a digital form by an analog-to-digital converter. A digital signal from the analog-to-digital converter may be a time-domain PPG signal fed to the processor 280. An output of an accelerometer may also be converted to a digital form by using the analog-to-digital converter. The processor 280 may receive a digital signal from the optical sensor, read the digital signal from an accelerometer, and process these signals to provide the heart rate or a wearing status output signal to a storage device, a visual display, an audible signal, a touchscreen, or another output indicator.

The wrist strap 200 includes an optical sensor and a motion sensor, the wrist strap can include another additional sensor. Specifically, the optical sensor may include an ambient optical sensor and a proximity sensor. The ambient optical sensor may adjust luminance of the display panel 241 based on brightness of ambient light. The proximity sensor may turn off the display panel 241 and/or backlight when the wrist strap 200 moves to an ear. As one type of the motion sensor, an accelerometer sensor may detect values of acceleration in various directions (usually on three axes). The accelerometer sensor may detect a value and a direction of gravity when the accelerometer sensor is in a static status, and may be applied to a function related to vibration recognition (such as a pedometer and a knock), and the like. Other sensors such as a gyroscope, a barometer, a hygrometer, a thermometer, and an infrared sensor may be further configured in the wrist strap 200. Details are not described herein.

The wrist strap 200 may further include the buzzer 252, and the buzzer 252 may generate vibration based on an instruction of the processor 280.

The audio circuit 260 may provide an audio interface between the user and the wrist strap 200. The audio circuit 260 may convert received audio data into a signal and transmit the signal to a speaker 261, and the speaker 261 converts the signal into a sound signal for output. In addition, a microphone converts a collected sound signal into a signal, and the audio circuit 260 receives the signal, converts the signal into audio data, and outputs the audio data to the RF circuit 210, to send the audio data to, for example, a mobile phone, or outputs the audio data to the memory 220 for further processing.

The I/O subsystem 270 is configured to control an external input/output device, and may include the another input device controller 271, a sensor controller 272, and a display controller 273. Optionally, one or more another input control device controllers 271 receive a signal from the another input device 230 and/or send a signal to the another input device 230. The another input device 230 may include a physical button (a pressing button, a rocker button, and the like), a dial pad, a slider switch, a joystick, a click scroll wheel, or an optical mouse (the optical mouse is a touch-sensitive surface that does not display a visible output, or an extension of a touch-sensitive surface formed by a touchscreen). It should be noted that the another input device controller 271 may be connected to any one or more of the foregoing devices. The display controller 273 in the I/O subsystem 270 receives a signal from the touchscreen 240 and/or sends a signal to the touchscreen 240. After the touchscreen 240 detects the user input, the display controller 273 converts the detected user input into interaction with a user interface object displayed on the touchscreen 240, to implement human-machine interaction. The sensor controller 272 may receive and/or send a signal from/to one or more sensors 251.

The processor 280 is a control center of the wrist strap 200, uses various interfaces and lines to connect each part of the entire mobile phone, and executes various functions and processes data of the wrist strap 200 by running or executing software programs and/or modules stored in the memory 220 and invoking data stored in the memory 220, to perform overall monitoring on the mobile phone. Optionally, the processor 280 may include one or more processing units. Preferably, an application processor and a modem processor may be integrated into the processor 280. The application processor mainly processes an operating system, a user interface, an application program, or the like. The modem processor mainly processes wireless communication. It may be understood that, alternatively, the modem processor may not be integrated in the processor 280.

The wrist strap 200 further includes the power supply 290 (such as a battery) supplying power to the components. Preferably, the power supply may be logically connected to the processor 280 by using a power management system, to implement functions such as management of charging, discharging, and energy consumption by using the power management system.

Although not shown, the wrist strap 200 may further include a camera, a Bluetooth module, and the like. Details are not described herein.

Modules stored in the memory 220 may include an operating system, a contact/motion module, an graphics module, an application program, and the like.

The contact/motion module is configured to: detect contact between an object or a finger and the touchscreen 240 or a tap-type touch rotatable device, capture a speed (direction and magnitude) and an acceleration (magnitude or direction change) of the contact, and determine a type of a contact event. For example, a module for detecting a plurality of contact events, sometimes a gesture is combined with an element in the user interface to implement some operations such as finger pinching/depinching (pinching/depinching).

The graphics module is configured to render and display a graphic on a touchscreen or another display. The graphic includes a web page, an icon, a digital image, a video, and an animation.

The application program may include contacts, a telephone, a video conference, an email client, instant messaging, personal motion, a camera, image management, a video player, a music player, a calendar, a plug-in (for example, weather, stock, a calculator, a clock, and a dictionary), a customized plug-in, searching, a note, a map, an online video, and the like.

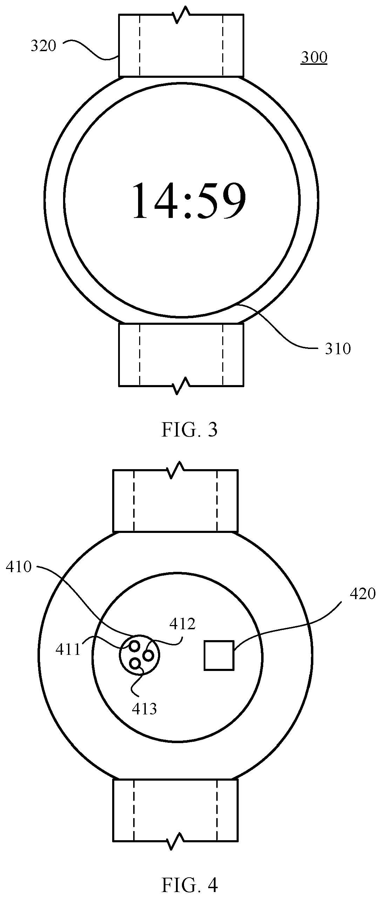

The following further describes this application by using an example in which a smartwatch is used as a wrist strap 200. FIG. 3 is a schematic structural diagram of a smartwatch according to an embodiment of this application. As shown in FIG. 3, the smartwatch 300 includes a watch face 310 and a watch strap 320. A front surface of the watch face 310 includes a display screen 311. The display screen 311 is configured to display information, for example, time, a motion status, a health status, or a wearing status. As shown in FIG. 4, FIG. 6, or FIG. 8, an optical transmitter and an optical sensor are disposed on a back surface of the watch face 310. The smartwatch may be worn on a wrist by using the watch strap 320. In this case, the back surface of the watch face 310 is attached to skin.

In this embodiment of this application, a preset optical path used to measure a wearing status of a user may be formed by a same optical transmitter and a same optical sensor, optical transmitters at different positions and a same optical sensor, a same optical transmitter and optical sensors at different positions, or optical transmitters and optical sensors at different positions. The smartwatch is used as an example to further describe a preset optical path of a wrist strap in this embodiment of the present invention. In an example, a preset optical path that may be formed by the same optical transmitter and the same optical sensor may generate lights of a plurality of colors. For example, the optical transmitter may include a plurality of light emitting elements, and each light emitting element transmits a light of a different light color.

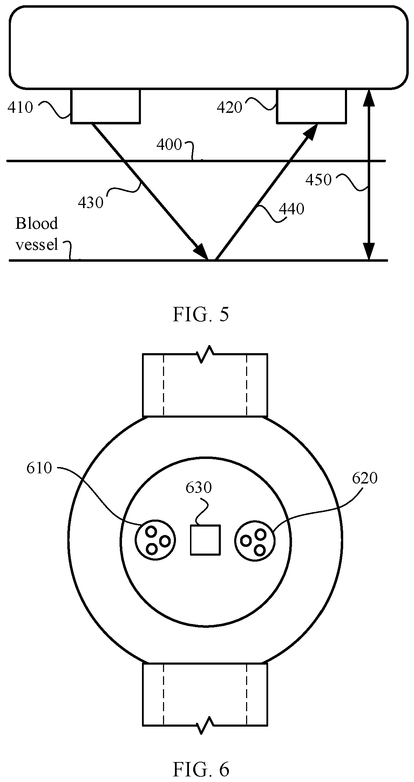

As shown in FIG. 4, the smartwatch includes an optical transmitter 410 and an optical sensor 420. Light emitting elements of the optical transmitter 410 include a green LED 411, a red LED 412, and an infrared LED 413. The optical transmitter 410 may send a green light, a red light, and an infrared light respectively by using the green LED 411, the red LED 412, and the infrared LED 413. The LED is merely an example of the light emitting element, and the LED may also be another light emitting element such as a VCSEL. With reference to FIG. 5, one or more of the green light, the red light, and the infrared light may be sent to a wrist direction by using the optical transmitter 410. A reflected light 440 obtained after a light 430 sent by the optical transmitter 410 is reflected by the wrist is received by the optical sensor 420. This process introduces noise 450. The noise 450 is affected by external ambient light intensity, external ambient temperature, human skin color depth, human skin roughness, human skin moisture content, human skin fat content, and the like, and is affected by the optical transmitter 410, the optical sensor 420, and skin 400. Lights of at least two light colors are determined by using the green LED 411, the red LED 412, the infrared LED 413, and the optical sensor 420, and the wearing status of the wrist strap is determined based on optical signal features of the at least two light colors. This can reduce impacts of the external ambient temperature, the human skin color depth, the human skin roughness, the human skin moisture content, the human skin fat content, and the like, and can better reflect impact of the external ambient light intensity, so that a determining result of the wearing status is more accurate.

In another example, a plurality of preset optical paths may be formed by the optical transmitters at different positions and the same optical sensor.

As shown in FIG. 6, the smartwatch includes an optical transmitter 610, an optical transmitter 620, and an optical sensor 630. A light emitting element of each optical transmitter may include a green LED, a red LED, and an infrared LED. The optical transmitter 610 and the optical sensor 630, and the optical transmitter 620 and the optical sensor 630 may separately form a preset optical path.

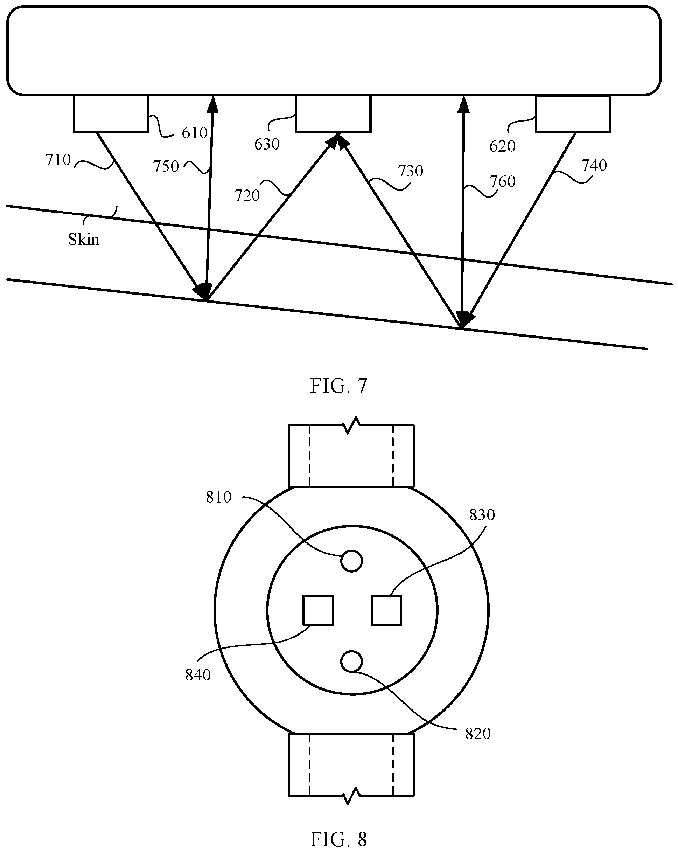

With reference to FIG. 7, a reflected light 720 that is obtained after a light 710 sent by using the optical transmitter 610 is reflected by the wrist is received by the optical sensor 630, and noise 750 is introduced. A reflected light 740 that is obtained after a light 730 sent by using the optical transmitter 620 is reflected by the wrist is received by the optical sensor 630, and noise 760 is introduced. Sending the light by using the optical transmitter 610 and receiving the light by using the optical sensor 630, and sending the light by using the optical transmitter 620 and receiving the light by using the optical sensor 630 are two different optical paths, and introduced noise 750 and 760 are different due to insufficient wearing. Lights on at least two preset optical paths are determined by using the optical transmitter 610, the optical sensor 630, the optical transmitter 620, and the optical sensor 630, and the wearing status of the wrist strap is determined based on the optical signal features at the two positions. This can reduce an error generated in determining the wearing status because a difference in distances between the watch face and the skin caused by wearing the wrist strap too loosely, and however, a distance between the optical transmitter in a single position and the skin is relatively short, and can more comprehensively determine noise introduced by a plurality of positions of the wrist strap, so that the determining result of the wearing status is more accurate. Further, the optical transmitter 610 and the optical sensor 630, and the optical transmitter 620 and the optical sensor 630 may be respectively corresponding to a plurality of light colors.

In another example, the optical transmitters at different positions and the optical sensors at different positions may form different preset optical paths.

As shown in FIG. 8, the smartwatch includes an optical transmitter 810, an optical transmitter 820, an optical sensor 830, and an optical sensor 840. Each optical transmitter may include a green LED, a red LED, and an infrared LED. The optical transmitter 810 and the optical sensor 830, and the optical transmitter 820 and the optical sensor 840 may separately form a preset optical path.

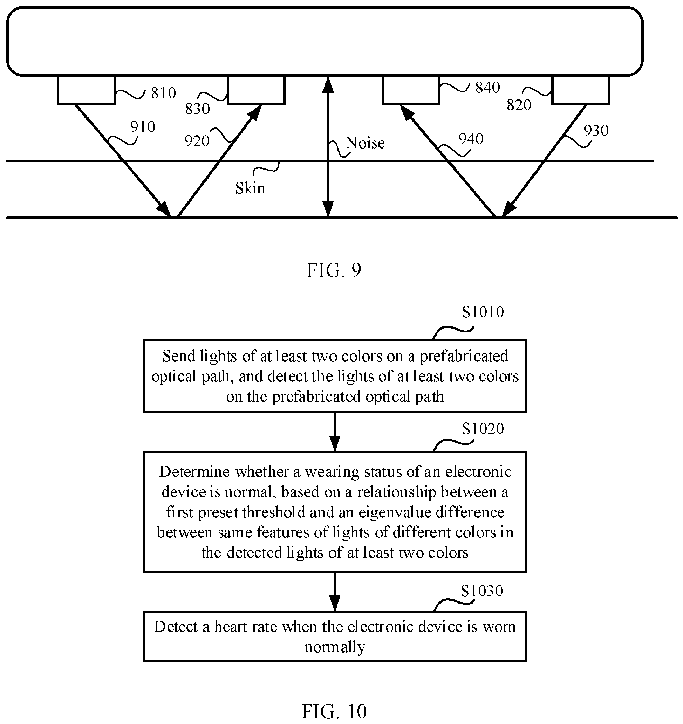

With reference to FIG. 9, a reflected light 920 that is obtained after a light 910 sent by using the optical transmitter 810 is reflected by the wrist is received by the optical sensor 830. A reflected light 940 obtained after a light 930 sent by using the optical transmitter 820 is reflected by the wrist is received by the optical sensor 840. Sending the light by using the optical transmitter 810 and receiving the light by using the optical sensor 930, and sending the light by using the optical transmitter 920 and receiving the light by using the optical sensor 940 are two different optical paths Lights on at least two preset optical paths are determined by using the optical transmitter 810, the optical sensor 830, the optical transmitter 820, and the optical sensor 840, and the wearing status of the wrist strap is determined based on the optical signal features at the two positions. This can reduce an error generated in determining the wearing status because a difference in distances between the watch face and the skin caused by wearing the wrist strap too loosely, and however, a distance between the optical transmitter in a single position and the skin is relatively short, and can more comprehensively determine noise introduced by a plurality of positions of the wrist strap, so that the determining result of the wearing status is more accurate. Further, the optical transmitter 810 and the optical sensor 830, and the optical transmitter 820 and the optical sensor 840 may respectively determine a plurality of light colors. The more preset optical paths and the more light colors, the more accurate obtained determining result.

The following further describes the embodiments of the present invention with reference to the accompanying drawings.

FIG. 10 is a schematic flowchart of a method for detecting a heart rate according to an embodiment of the present invention. As shown in FIG. 10, the method specifically includes the following steps.

S1010. An electronic device sends lights of at least two colors on a preset optical path, and detects the lights of at least two colors on the preset optical path.

The preset optical path may be an optical path that is preset and that is used to detect a heart rate, and each optical path is formed by one optical transmitter and one optical sensor. During specific implementation of this embodiment of this application, there may be one or more preset optical paths. When there is one preset optical path, the preset optical path includes the lights of at least two colors. When there are a plurality of preset optical paths, each preset optical path includes lights of one or more colors. For example, two optical transmitters 610 and 620 at different positions shown in FIG. 6 and FIG. 7 may form two preset optical paths respectively with a same optical sensor 630. For another example, two optical transmitters 810 and 820 at different positions shown in FIG. 8 and FIG. 9 may form two preset optical paths respectively with two optical sensors 830 and 840 at different positions.

The lights of one or more colors include a red light, a green light, an infrared light, or the like. For example, the red light, the green light, or the infrared light may be sent by using an LED, a VCSEL, or the like. For example, the lights of at least two colors may be sent and received by the optical transmitter and the optical sensor shown in any one in FIG. 4 to FIG. 8.

The lights may be sent by the wrist strap to a direction corresponding to a wrist, and is received after being reflected by wrist skin and a blood vessel. A wearing status of the electronic device may be determined by using optical signal features of the lights of at least two colors. A difference of the features used to determine the wearing status of the electronic device includes one or more of the following: a difference between same features includes one or more of the following: an absolute value difference between optical signals of lights of different colors, an amplitude difference between optical signals of lights of different colors, a change rate difference between optical signals of lights of different colors, a root mean square difference between optical signals of lights of different colors, a spectral component difference between optical signals of lights of different colors, and the like.

In addition, signals corresponding to the lights of at least two colors in a PPG signal may be multiplexed to determine the wearing status, or dedicated lights of at least two colors may be used to determine the wearing status.

Signal features of the lights of at least two colors may be determined at least in the following manner.

In an example, the wrist strap may obtain, through detection by using a PPG module, PPG signals corresponding to the lights of at least two colors, determine PPG signal features based on the PPG signals obtained through measurement, and send the PPG signal features to the electronic device (for example, a mobile phone), so that the electronic device performs operations such as heart rate detection based on the PPG signal features. Based on this, step S1010 may be implemented by using the following steps.

The electronic device may receive the lights that is of at least two colors and that is sent by the wrist strap and that is detected on the preset optical path.

In another example, the wrist strap may obtain, through detection by using the PPG module, the PPG signals corresponding to the lights of at least two colors, and perform operations such as the heart rate detection based on the measured PPG signal feature.

S1020. The electronic device determines whether a wearing status of the electronic device is normal, based on a relationship between a first preset threshold and a difference between same features of lights of different colors in the detected lights of at least two colors.

The wearing status of the electronic device may be set in a plurality of cases based on an actual requirement. For example, the wearing status includes at least being worn too loosely and being worn normally. When the difference is less than the first preset threshold, the wearing status of the electronic device is being worn too loosely. When the difference is greater than the preset threshold, the wearing status of the electronic device is being worn normally. Optionally, the wearing status may further include being worn too tightly, not being worn, and the like. Alternatively, the wearing status may further include being worn correctly, not being worn correctly, and the like. In an example, the first preset threshold may be a range. When the difference exceeds the first preset threshold range, it indicates that the electronic device is worn too tightly. When the difference is less than the first preset threshold range, it indicates that the electronic device is worn too loosely. When the difference is within the first preset threshold range, it indicates that the electronic device is worn normally. In another example, the first preset threshold may include a plurality of gradients, and each gradient corresponds to one wearing level. For example, a status of being worn correctly may include three thresholds (a minimum threshold, an intermediate threshold, and a maximum threshold). The three thresholds may be divided into four gradients: a first gradient, a second gradient, a third gradient, and a fourth gradient. The first gradient is not worn, and in this case, the difference is less than the minimum threshold. The second gradient is worn too loosely, and in this case, the difference is greater than the minimum threshold and less than the intermediate threshold. The third gradient is worn normally, and in this case, the difference is greater than the intermediate threshold and less than the maximum threshold. The fourth gradient is worn too tightly, and in this case, the difference is greater than the maximum threshold.

In this embodiment of the present invention, determining the wearing status of the electronic device based on the detected lights of at least two colors may include at least the following manners.

In an example, a wearing status detection model may be pre-trained based on a machine learning technology, and the wearing status is output by using a feature of an optical signal as an input of the heart rate detection model.

In addition, the wearing status is output by using one or more of the optical signal feature combined with environment information, a motion status, and the like as an input of the wearing status detection model. A training sample of the wearing status detection model may be predetermined, or generated through simulation, or the like. In actual application, the environment information and the motion status may be determined based on information detected by a sensor on the wrist strap, or may be determined based on data provided by a third-party application.

In another example, the difference of the optical signals may further include: a difference between a transmitted light and a detected light that are corresponding to a same color and that are on a same preset optical path, a difference between lights of a same color on different preset optical paths, a difference between lights of different colors, and the like. The wearing status of the electronic device may be determined with reference to a relationship between the foregoing one or more differences and a second preset threshold, and a relationship between a difference between the lights of different colors in the detected lights of at least two colors and the first preset threshold.

A second threshold corresponding to the difference between the transmitted light and the detected light corresponding to the same color on the same preset optical path may be determined based on an optical signal feature threshold, and the optical signal feature threshold may be determined based on an actual requirement.

For example, an amplitude threshold of an optical signal whose transmitted light is the green light may be [0.5, 1.0], and an amplitude threshold of an optical signal whose transmitted light is the infrared light may be [0.4, 1.0].

A change rate threshold of the optical signal whose transmitted light is the green light may be [0.1, 0.5], and a change rate threshold of the optical signal whose transmitted light is the infrared light may be [0.2, 0.8].

A root mean square threshold of the optical signal whose transmitted light is the green light may be [0.1, 0.2], and a root mean square threshold of the optical signal whose transmitted light is the infrared light may be [0.1, 0.4].

A spectral component threshold of the optical signal whose transmitted light is the green light may be a proportion of f (0.5 to 3 Hz), which is [0.3, 0.10]. A spectral component threshold of the optical signal whose transmitted light is the infrared light may be a proportion of f (0.5 to 3 Hz), which is [0.3, 0.10]. The first preset threshold may be implemented in a plurality of manners based on different compositions of features of determining the wearing status of the electronic device.

In an example, the first preset threshold may be a threshold corresponding to a single feature, and the threshold corresponding to the single feature may be a difference between same features of lights of different colors. For example, an amplitude difference threshold between a red light and a green light.

In another example, the first preset threshold may be a proportion of features between which a difference is normal in a plurality of features. For example, a first weight corresponding to each feature may be predetermined, a plurality of features are multiplied by corresponding weights, and a proportion of features between which the difference is normal in the plurality of features that are multiplied by the weights may be determined. Each feature corresponds to one third threshold, and features between which a difference falls within a corresponding third preset threshold range are normal features. Then, the wearing status of the electronic device is determined based on the proportion of features between which a difference is nomal in the plurality of features.

For example, an optical signal feature used to determine the wearing status corresponds to two features, and first weights of the two features are both 0.5. In this case, a first threshold may be 51 percent. When both the two features are normal, it is determined that the wearing status of the electronic device is normal. When either of the two features is abnormal, it is determined that the electronic device is abnormal.

In another example, the first preset threshold may alternatively be a tightness value. For example, a first weight corresponding to each feature may be predetermined, and a wearing tightness value is output based on a relationship between an optical signal feature and a signal feature threshold of a transmitted light corresponding to the optical signal feature. For example, when the optical signal feature is within the threshold, a corresponding tightness value is 0, when the optical signal feature is less than the threshold, a corresponding tightness value is -1, and when the optical signal feature is greater than the threshold, an output value is 1. A product of the wearing tightness value corresponding to each feature used for wearing status detection and the first weight corresponding to the wearing tightness value is accumulated, to obtain a result value through calculation. The wearing status is determined based on a relationship between the result value and the first threshold. For example, the first threshold may be [-0.5, 0.5]. If the result value is greater than the first threshold, the wearing status is too loose. If the result value is less than the first threshold, the wearing status is too tight.

When the wearing status is determined, different first weights are set based on different motion statuses. For example, the user tends to wear the electronic device tightly in the motion status, to detect the heart rate more accurately. A dynamic component of the optical signal is mainly monitored, and weights of features such as a change rate of the optical signal, a root mean square of the optical signal, and a spectral component of the optical signal are high. The user tends to wear the electronic device loosely to improve comfort in a static status (for example, sleeping at night). Focus on monitoring a static component. Weights of an absolute value and amplitude of the optical signal are high. Based on this, step 1020 may specifically include the following steps. A current motion status of the user and weights of a plurality of features corresponding to the current motion status are determined. A higher requirement of a same motion status on feature stability indicates a higher weight. The plurality of features are multiplied by weights corresponding to the plurality of features, and a proportion of features between which a difference is nomal in the plurality of features that are multiplied by weights is determined. Each feature corresponds to one third threshold, and features between which a difference falls within a corresponding third preset threshold range are normal features. When the proportion of features between which a difference is nomal in the plurality of features is greater than the first preset threshold, it is determined that the electronic device is worn normally.

The first weight corresponding to each feature may be further determined with reference to surrounding environment information, a motion status, and the like. For example, it is determined that when the user is in a motion status, a first weight of a feature corresponding to a green light is greater than a feature corresponding to an infrared light. When the user is in a static status, the first weight of the feature corresponding to the green light is less than or equal to the feature corresponding to the infrared light. When an ambient light is relatively dim, a first weight of the feature corresponding to the infrared light is greater than the feature corresponding to the green light. When the ambient light is relatively bright, the first weight of the feature corresponding to the infrared light is less than or equal to the feature corresponding to the green light. The surrounding environment information may be determined by using a sensor on the wrist strap. For example, whether the user is in the motion status or the static status may be determined based on data measured by an accelerometer. Based on local time information, it is determined whether detection is performed in daytime or nighttime, to determine ambient light intensity. Alternatively, light intensity may be measured by using a light intensity measuring device.

In another example, whether the electronic device is worn normally may be further determined based on a difference between lights of a same color on different preset optical paths. For example, a signal obtained after a transmitted light of each light color is reflected by the wrist is from two preset optical paths at different positions, and path lengths of the two preset optical paths may be the same.

The difference between the lights of a same color on different preset optical paths may include one or more of the following:
An signal absolute value difference of the lights of a same color on different preset optical paths, for example, a ratio of signal absolute values of the lights of a same color on different preset optical paths; a signal amplitude difference of the lights of a same color on different preset optical paths; a change rate difference of the lights of a same color on different preset optical paths; a signal root mean square difference of the lights of a same color on different preset optical paths; a signal spectral component difference of the lights of a same color on different preset optical paths; and the like.

The wearing status of the electronic device may be determined based on a relationship between a second preset threshold and a difference between lights of a same color on different preset optical paths. A second threshold corresponding to a transmitted light of each color light may be different. For example, a second threshold of a signal absolute value ratio corresponding to the green light may be [0.5, 1.5], and a second threshold of a signal absolute value ratio corresponding to the infrared light may be [0.3, 1.7].

In addition, a wearing tightness value may be further output based on a relationship between each difference and a second threshold corresponding to the difference. For example, when the difference falls within a second threshold range, a corresponding tightness value is 0, when the difference is less than the second threshold, a corresponding tightness value is -1, and when the difference is greater than the second threshold, an output value is 1. A product of the wearing tightness value corresponding to each difference and a corresponding first weight is accumulated, to obtain a result value through calculation. The wearing status is determined based on a relationship between the result value and a third threshold. For example, the third threshold may be [-0.5, 0.5]. If the result value is greater than a third threshold, the wearing status is too loose. If the result value is less than the third threshold, the wearing status is too tight.

Because there may be a plurality of differences of the optical signal features, the plurality of features may be combined to determine a final wearing status. For example, the wearing tightness value is output based on the relationship between each difference and the second threshold corresponding to each difference, a product of the wearing tightness value corresponding to each difference and a first weight and a second weight corresponding to each difference is accumulated, to obtain a result value through calculation. The wearing status is determined based on a relationship between the result value and a fourth threshold.

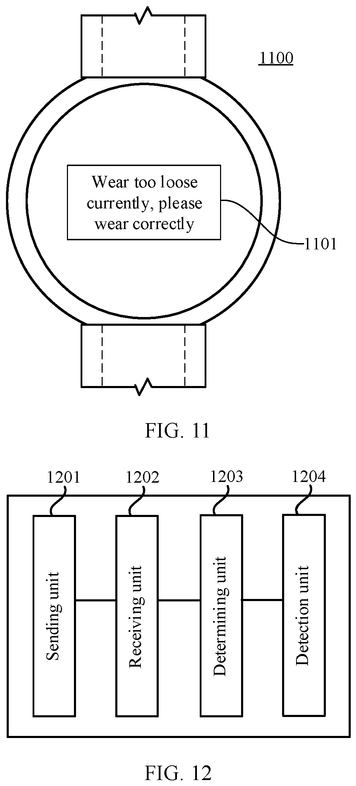

After the wearing status of the wrist strap is determined, when the wearing status is abnormal, a prompt is generated. The wearing status may be prompted in a form of an image and a text, for example, in a form of a system notification, or in an interface of an application such as heart rate measurement and motion measurement that the wearing status does not meet a requirement. For example, as shown in FIG. 11, when the wearing status is too loose, the wearing status "wear too loose currently, please wear correctly 1101" of text information may be displayed on a display screen of the electronic device.

Further, after an instruction of the user is received, a guide video or a guide picture and text for correctly wearing may be further displayed. Alternatively, when the status of the wrist strap does not meet the requirement, a vibration prompt may be provided, for example, the vibration prompt is provided by using a buzzer. Alternatively, when the status of the wrist strap does not meet the requirement, a voice prompt may be provided, for example, the voice prompt is provided by using a speaker.

S1130. When the electronic device is worn normally, the electronic device detects a heart rate.

After the wearing status of the wrist strap is determined, the heart rate is detected. This can reduce an error caused by interference introduced by an abnormal wearing status, and improve accuracy and reliability of the heart rate detection.

In addition, heart rate measurement may be further performed with reference to the wearing status of the electronic device. For example, a heart rate detection result may be optimized based on an error introduced by the wearing status of the electronic device. For example, a heart rate measurement algorithm may be optimized based on the wearing status of the wrist strap, so that impact of the wearing status is considered in a heart rate measurement calculation process, to improve heart rate measurement precision.

According to this embodiment of the present invention, the wearing status of the wrist strap can be determined by using the lights of different colors, so as to reduce impact posed on a determining result by a light of a single color that is easily interfered. After it is determined that the wrist strap is worn normally, a heart rate is detected. This improves accuracy of the heart rate detection. The wearing status is detected by using the lights on at least two preset optical paths, so that an anti-interference capability of the heart rate detection can be further increased, and a detection result is more accurate. The PPG signal is multiplexed for the heart rate detection. which is more convenient and cost-effective.

FIG. 12 is a schematic structural diagram of an apparatus for detecting a heart rate according to an embodiment of the present invention. The apparatus is configured to implement the method shown in FIG. 10. As shown in FIG. 12, the apparatus includes:
a sending unit 1201, configured to send lights of at least two colors on a preset optical path;
a receiving unit 1202, configured to detect the lights of at least two colors on the preset optical path;
a determining unit 1203, configured to determine whether a wearing status of the electronic device is normal, based on a relationship between a first preset threshold and a difference between same features of lights of different colors in the detected lights of at least two colors; and
a detection unit 1204, configured to detect a heart rate when the electronic device is worn normally.

In an embodiment, there are at least two preset optical paths, and each preset optical path includes lights of one or more colors.

In another embodiment, the lights of at least two colors are PPG signals.

In another embodiment, the determining unit 1203 is specifically configured to: determine whether the wearing status of the electronic device is normal, with reference to a relationship between a second preset threshold and a difference between same features that are lights of a same color in the detected lights of at least two colors and that are on different preset optical paths, and the relationship between the first preset threshold and the eigenvalue difference between same features of lights of different colors in the lights of at least two colors.

In another embodiment, the eigenvalue difference of the same features includes one or more of the following: an absolute value difference between optical signals, an amplitude difference between optical signals, a change rate difference between optical signals, a root mean square difference between optical signals, and a spectral component difference between optical signals.

In another embodiment, the determining unit 1203 is specifically configured to: determine a current motion status of a user and weights of a plurality of features corresponding to the current motion status, where a higher requirement of a same motion status on feature eigenvalue stability indicates a higher weight;
respectively multiply the plurality of features by the weights corresponding to the plurality of features, and determine a proportion of features between which a difference is nomal in the plurality of features that are multiplied by the weights, where each feature corresponds to one third threshold, and features between which a difference falls within a corresponding third preset threshold range are normal features; and
when the proportion of features between which a difference is nomal in the plurality of features is greater than the first preset threshold, determine that the electronic device is worn normally.

In another optional implementation, the wearing status includes at least being worn too loosely and being worn normally. When the eigenvalue difference is less than the first preset threshold, the wearing status of the electronic device is being worn too loosely. When the eigenvalue difference is greater than the first preset threshold, the wearing status of the electronic device is being worn normally. Optionally, the wearing status may further include being worn too tightly, not being worn, and the like. Alternatively, the wearing status may further include being worn correctly, not being worn correctly, and the like.

In another embodiment, the apparatus further includes:
a prompting unit, configured to prompt the user when the electronic device is worn abnormally.

In another embodiment, the detection unit is further configured to optimize a heart rate detection result based on a magnitude of an error introduced by the wearing status of the electronic device.

All or some of the foregoing embodiments of the present invention may be implemented by using software, hardware, firmware, or any combination thereof. When software is used to implement the embodiments, the embodiments may be implemented completely or partially in a form of a computer program product. The computer program product includes one or more computer instructions. When the computer program instructions are loaded and executed on the computer, the procedure or functions according to the embodiments of the present invention are all or partially generated. The computer may be a general-purpose computer, a dedicated computer, a computer network, or other programmable apparatuses. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line (digital subscriber line, DSL)) or wireless (for example, infrared, radio, or microwave) manner. The computer-readable storage medium may be any usable medium accessible by a computer, or a data storage device, such as a server or a data center, integrating one or more usable media. The usable medium may be a magnetic medium (for example, a floppy disk, a hard disk, or a magnetic tape), an optical medium (for example, a DVD), a semiconductor medium (for example, a solid-state drive), or the like.

The foregoing descriptions are merely example specific implementations of this application, but are not intended to limit the protection scope of this application. The scope of the invention is defined by the appended claims.

## Claims

1. An electronic device (100) for detecting a heart rate, comprising:
• one or more optical transmitters (141) configured to send lights of two colors on a preset optical path;
• one or more optical sensors (142) configured to detect the lights on the preset optical path;
• one motion sensor configured to detect motion data;
• a processor (110) connected to the motion sensor, the one or more optical sensors and the one or more optical transmitters; and
• a prompter (150);
wherein the processor (110) is configured to determine whether a wearing status of the electronic device (100) is normal or abnormal, wherein, for determining whether the wearing status of the electronic device (100) is normal or abnormal, the processor (110) is configured to:
∘ derive, for the detected light of each color among the two colors, a plurality of feature values, wherein each feature value among the plurality of feature values is of a different feature among a plurality of different features;
o determine a current motion status of a user based on the motion data,
∘ determine, for the light of each color among the two colors, a weight for each feature among the plurality of different features corresponding to the current motion status, wherein a higher requirement of a same motion status on feature stability indicates a higher weight;
∘ multiply, for the light of each color among the two colors, each feature value among the plurality of feature values by its corresponding weight,
∘ determine, for each feature among the plurality of different features, a difference between the corresponding feature values of the lights of the different colors among the two colors that have been multiplied with its corresponding weights;
∘ determine a proportion of said features whose difference is normal among the plurality of different features, wherein each feature corresponds to one preset threshold range, and a feature whose difference falls within the corresponding preset threshold range is a normal features; and
o when the proportion of said features whose difference is normal among the plurality of different features is greater than a first preset threshold, determine, as the wearing status of the electronic device (100), that the electronic device (100) is worn normally, otherwise, determine, as the wearing status of the electronic device (100), that the electronic device (100) is worn abnormally,
• wherein:
∘ when it is determined that the electronic device (100) is worn normally, the electronic device (100) is configured to detect the heart rate of the user, and
∘ when it is determined that the electronic device is worn abnormally, the prompter (150) is configured to prompt the user.

2. The electronic device (100) according to claim 1, wherein there are at least two preset optical paths, each optical path comprises one optical transmitter (141) and one optical sensor (142), and each optical transmitter (141) sends lights of one or more colors on the preset optical path.

3. The electronic device (100) according to claim 1 or 2, wherein the one or more optical transmitters (141) and the one or more optical sensors (142) multiplex an optical transmitter and an optical sensor in a PPG module.

4. The electronic device (100) according to any one of claims 1 to 3, wherein the processor (110) is configured to rely additionally for the determination whether the wearing status of the electronic device (100) is normal on a relationship between a second preset threshold and a difference of feature values of a same feature that are of a same color in the detected lights of the two colors and that are on different preset optical paths.

5. The electronic device (100) according to any one of claims 1 to 4, wherein the difference between the feature values is any one of the following: an absolute value difference between optical signals of lights of different colors, an amplitude difference between optical signals of lights of different colors, a change rate difference between optical signals of lights of different colors, a root mean square difference between optical signals of lights of different colors, and a spectrum component difference between optical signals of lights of different colors.

6. A method for detecting a heart rate performed by an electronic device (100), wherein the electronic device (100) comprises:
• one or more optical transmitters (141);
• one or more optical sensors (142);
• one motion sensor configured to detect motion data;
• a processor (110) connected to the motion sensor, the one or more optical sensors and the one or more optical transmitters; and
• a prompter (150);
wherein the method comprises the following steps:
∘ sending (S1010), by the one or more optical transmitters (141), lights of two colors on a preset optical path;
∘ detecting (S1010), by the one or more optical sensors (142), the lights on the preset optical path;
∘ determining, by the processor (110), whether a wearing status of the electronic device (100) is normal or abnormal, wherein the determining whether the wearing status of the electronic device (100) is normal or abnormal comprises:
- deriving, for the detected light of each color among the two colors, a plurality of feature values, wherein each feature value among the plurality of feature values is of a different feature among a plurality of different features;
- determining a current motion status of a user based on the motion data,
- determining, for the light of each color among the two colors, a weight for each feature among the plurality of different features corresponding to the current motion status, wherein a higher requirement of a same motion status on feature stability indicates a higher weight;
- multiplying, for the light of each color among the two colors, each feature value among the plurality of feature values by its corresponding weight,
- determining, for each feature among the plurality of different features, a difference between the corresponding feature values of the lights of the different colors among the two colors that have been multiplied with its corresponding weights;
- determining a proportion of said features whose difference is normal among the plurality of different features, wherein each feature corresponds to one preset threshold range, and a feature whose difference falls within the corresponding preset threshold range is a normal features; and
- when the proportion of said features whose difference is normal among the plurality of different features is greater than a first preset threshold, determining, as the wearing status of the electronic device (100), that the electronic device (100) is worn normally, otherwise, determining, as the wearing status of the electronic device (100), that the electronic device (100) is worn abnormally,
• wherein:
∘ when it is determined that the electronic device (100) is worn normally, detecting (S1030), by the electronic device (100), the heart rate of the user, and
∘ when it is determined that the electronic device is worn abnormally, prompting, by the prompter (150), the user.

7. The method according to claim 6, wherein there are at least two preset optical paths, and each preset optical path comprises lights of one or more colors.

8. The method according to claim 6 or 7, wherein the lights of two colors are PPG signals.

9. The method according to any one of claims 6 to 8, wherein the determination whether the wearing status of the electronic device (100) is normal relies additionally on a relationship between a second preset threshold and a difference of feature values of a same feature that is of a same color in the detected lights of two colors and that are on different preset optical paths..

10. The method according to any one of claims 6 to 9, wherein the difference between feature values of the same feature is any one of the following: an absolute value difference between optical signals, an amplitude difference between optical signals, a change rate difference between optical signals, a root mean square difference between optical signals, and a spectrum component difference between optical signals.

11. A computer program product comprising computer-readable instructions, wherein, when a computer reads and executes the computer-readable instructions, the computer is caused to perform the method according to any one of claims 6 to 10.

## Patentansprüche

1. Elektronische Vorrichtung (100) zum Detektieren einer Herzfrequenz, umfassend:
• einen oder mehrere optische Sender (141), die dazu konfiguriert sind, Lichter zweier Farben auf einem voreingestellten optischen Pfad zu senden;
• einen oder mehrere optische Sensoren (142), die dazu konfiguriert sind, die Lichter auf dem voreingestellten optischen Pfad zu detektieren;
• einen Bewegungssensor, der dazu konfiguriert ist, Bewegungsdaten zu detektieren;
• einen Prozessor (110), der mit dem Bewegungssensor, dem einen oder den mehreren optischen Sensoren und dem einen oder den mehreren optischen Sendern verbunden ist; und
• einen Hinweisgeber (150);
wobei der Prozessor (110) dazu konfiguriert ist, zu bestimmen, ob ein Tragezustand der elektronischen Vorrichtung (100) normal oder anormal ist, wobei der Prozessor (110) zum Bestimmen, ob der Tragezustand der elektronischen Vorrichtung (100) normal oder anormal ist, zu Folgendem konfiguriert ist:
∘ Ableiten, für das detektierte Licht jeder Farbe unter den zwei Farben, einer Vielzahl von Merkmalswerten, wobei jeder Merkmalswert unter der Vielzahl von Merkmalswerten zu einem unterschiedlichen Merkmal unter einer Vielzahl von unterschiedlichen Merkmalen gehört;
∘ Bestimmen eines aktuellen Bewegungszustands eines Benutzers basierend auf den Bewegungsdaten,
∘ Bestimmen, für das Licht jeder Farbe unter den zwei Farben, einer Gewichtung für jedes Merkmal unter der Vielzahl von unterschiedlichen Merkmalen, die dem aktuellen Bewegungszustand entsprechen, wobei eine höhere Anforderung eines gleichen Bewegungszustands an eine Merkmalsstabilität eine höhere Gewichtung bedeutet;
o Multiplizieren, für das Licht jeder Farbe unter den zwei Farben, jedes Merkmalswerts unter der Vielzahl von Merkmalswerten mit seiner entsprechenden Gewichtung,
∘ Bestimmen, für jedes Merkmal unter der Vielzahl von unterschiedlichen Merkmalen, einer Differenz zwischen den entsprechenden Merkmalswerten der Lichter der unterschiedlichen Farben unter den zwei Farben, die mit ihren entsprechenden Gewichtungen multipliziert wurden;
∘ Bestimmen eines Anteils der Merkmale, deren Differenz normal ist, unter der Vielzahl von unterschiedlichen Merkmalen, wobei jedes Merkmal einem voreingestellten Schwellenwertbereich entspricht und ein Merkmal, dessen Differenz innerhalb des entsprechenden voreingestellten Schwellenwertbereichs liegt, ein normales Merkmal ist; und
∘ wenn der Anteil der Merkmale, deren Differenz normal ist, unter der Vielzahl von unterschiedlichen Merkmalen größer als ein erster voreingestellter Schwellenwert ist, Bestimmen, als den Tragezustand der elektronischen Vorrichtung (100), dass die elektronische Vorrichtung (100) normal getragen wird, ansonsten Bestimmen, als den Tragezustand der elektronischen Vorrichtung (100), dass die elektronische Vorrichtung (100) anormal getragen wird,
• wobei:
o wenn bestimmt wird, dass die elektronische Vorrichtung (100) normal getragen wird, die elektronische Vorrichtung (100) dazu konfiguriert ist, die Herzfrequenz des Benutzers zu detektieren, und
∘ wenn bestimmt wird, dass die elektronische Vorrichtung anormal getragen wird, der Hinweisgeber (150) dazu konfiguriert ist, dem Benutzer einen Hinweis zu geben.

2. Elektronische Vorrichtung (100) nach Anspruch 1, wobei mindestens zwei voreingestellte optische Pfade vorhanden sind, wobei jeder optische Pfad einen optischen Sender (141) und einen optischen Sensor (142) umfasst und jeder optische Sender (141) Licht einer oder mehrerer Farben auf dem voreingestellten optischen Pfad sendet.

3. Elektronische Vorrichtung (100) nach Anspruch 1 oder 2, wobei der eine oder die mehreren optischen Sender (141) und der eine oder die mehreren optischen Sensoren (142) einen optischen Sender und einen optischen Sensor in einem PPG-Modul multiplexen.

4. Elektronische Vorrichtung (100) nach einem der Ansprüche 1 bis 3, wobei der Prozessor (110) dazu konfiguriert ist, sich für die Bestimmung, ob der Tragezustand der elektronischen Vorrichtung (100) normal ist, zusätzlich auf eine Beziehung zwischen einem zweiten voreingestellten Schwellenwert und einer Differenz von Merkmalswerten eines gleichen Merkmals zu stützen, die zu einer gleichen Farbe in den detektierten Lichtern der zwei Farben gehören und die sich auf unterschiedlichen voreingestellten optischen Pfaden befinden.

5. Elektronische Vorrichtung (100) nach einem der Ansprüche 1 bis 4, wobei die Differenz zwischen den Merkmalswerten ein beliebiges des Folgenden ist: eine absolute Wertdifferenz zwischen optischen Signalen von Lichtern unterschiedlicher Farben, eine Amplitudendifferenz zwischen optischen Signalen von Lichtern unterschiedlicher Farben, eine Änderungsratendifferenz zwischen optischen Signalen von Lichtern unterschiedlicher Farben, eine quadratische Mittelwertdifferenz zwischen optischen Signalen von Lichtern unterschiedlicher Farben und eine Spektralkomponentendifferenz zwischen optischen Signalen von Lichtern unterschiedlicher Farben.

6. Verfahren zum Detektieren einer Herzfrequenz, durchgeführt durch eine elektronische Vorrichtung (100), wobei die elektronische Vorrichtung (100) Folgendes umfasst:
• einen oder mehrere optische Sender (141);
• einen oder mehrere optische Sensoren (142);
• einen Bewegungssensor, der dazu konfiguriert ist, Bewegungsdaten zu detektieren;
• einen Prozessor (110), der mit dem Bewegungssensor, dem einen oder den mehreren optischen Sensoren und dem einen oder den mehreren optischen Sendern verbunden ist; und
• einen Hinweisgeber (150);
wobei das Verfahren die folgenden Schritte umfasst:
∘ Senden (S1010), durch den einen oder die mehreren optischen Sender (141), von Lichtern zweier Farben auf einem voreingestellten optischen Pfad;
∘ Detektieren (S1010), durch den einen oder die mehreren optischen Sensoren (142), der Lichter auf dem voreingestellten optischen Pfad;
o Bestimmen, durch den Prozessor (110), ob ein Tragezustand der elektronischen Vorrichtung (100) normal oder anormal ist, wobei das Bestimmen, ob der Tragezustand der elektronischen Vorrichtung (100) normal oder anormal ist, Folgendes umfasst:
- Ableiten, für das detektierte Licht jeder Farbe unter den zwei Farben, einer Vielzahl von Merkmalswerten, wobei jeder Merkmalswert unter der Vielzahl von Merkmalswerten zu einem anderen Merkmal unter einer Vielzahl von unterschiedlichen Merkmalen gehört;
- Bestimmen eines aktuellen Bewegungszustands eines Benutzers basierend auf den Bewegungsdaten,
- Bestimmen, für das Licht jeder Farbe unter den zwei Farben, einer Gewichtung für jedes Merkmal unter der Vielzahl von unterschiedlichen Merkmalen, die dem aktuellen Bewegungszustand entsprechen, wobei eine höhere Anforderung eines gleichen Bewegungszustands an eine Merkmalsstabilität eine höhere Gewichtung bedeutet;
- Multiplizieren, für das Licht jeder Farbe unter den zwei Farben, jedes Merkmalswerts unter der Vielzahl von Merkmalswerten mit seiner entsprechenden Gewichtung,
- Bestimmen, für jedes Merkmal unter der Vielzahl von unterschiedlichen Merkmalen, einer Differenz zwischen den entsprechenden Merkmalswerten der Lichter der unterschiedlichen Farben unter den zwei Farben, die mit ihren entsprechenden Gewichtungen multipliziert wurden;
- Bestimmen eines Anteils der Merkmale, deren Differenz normal ist, unter der Vielzahl von unterschiedlichen Merkmalen, wobei jedes Merkmal einem voreingestellten Schwellenwertbereich entspricht und ein Merkmal, dessen Differenz innerhalb des entsprechenden voreingestellten Schwellenwertbereichs liegt, ein normales Merkmal ist; und
- wenn der Anteil der Merkmale, deren Differenz normal ist, unter der Vielzahl von unterschiedlichen Merkmalen größer als ein erster voreingestellter Schwellenwert ist, Bestimmen, als den Tragezustand der elektronischen Vorrichtung (100), dass die elektronische Vorrichtung (100) normal getragen wird, ansonsten Bestimmen, als den Tragezustand der elektronischen Vorrichtung (100), dass die elektronische Vorrichtung (100) anormal getragen wird,
• wobei:
∘ wenn bestimmt wird, dass die elektronische Vorrichtung (100) normal getragen wird, Detektieren (S1030), durch die elektronische Vorrichtung (100), der Herzfrequenz des Benutzers und
∘ wenn bestimmt wird, dass die elektronische Vorrichtung anormal getragen wird, Geben eines Hinweises, durch den Hinweisgeber (150), an den Benutzer.

7. Verfahren nach Anspruch 6, wobei mindestens zwei voreingestellte optische Pfade vorhanden sind und jeder voreingestellte optische Pfad Lichter einer oder mehrerer Farben umfasst.

8. Verfahren nach Anspruch 6 oder 7, wobei die Lichter zweier Farben PPG-Signale sind.

9. Verfahren nach einem der Ansprüche 6 bis 8, wobei sich die Bestimmung, ob der Tragezustand der elektronischen Vorrichtung (100) normal ist, zusätzlich auf eine Beziehung zwischen einem zweiten voreingestellten Schwellenwert und einer Differenz von Merkmalswerten eines gleichen Merkmals stützt, das zu einer gleichen Farbe in den detektierten Lichtern zweier Farben gehört, und die sich auf unterschiedlichen voreingestellten optischen Pfaden befinden.

10. Verfahren nach einem der Ansprüche 6 bis 9, wobei die Differenz zwischen Merkmalswerten des gleichen Merkmals ein beliebiges des Folgenden ist: eine absolute Wertdifferenz zwischen optischen Signalen, eine Amplitudendifferenz zwischen optischen Signalen, eine Änderungsratendifferenz zwischen optischen Signalen, eine quadratische Mittelwertdifferenz zwischen optischen Signalen und eine Spektralkomponentendifferenz zwischen optischen Signalen.

11. Computerprogrammprodukt, umfassend computerlesbare Anweisungen, wobei, wenn ein Computer die computerlesbaren Anweisungen liest und ausführt, der Computer dazu veranlasst wird, das Verfahren nach einem der Ansprüche 6 bis 10 durchzuführen.

## Revendications

1. Dispositif électronique (100) de détection d'une fréquence cardiaque, comprenant :
• un ou plusieurs émetteurs optiques (141) configurés pour envoyer des lumières de deux couleurs sur un chemin optique prédéfini ;
• un ou plusieurs capteurs optiques (142) configurés pour détecter les lumières sur le chemin optique prédéfini ;
• un capteur de mouvement configuré pour détecter les données de mouvement ;
• un processeur (110) connecté au capteur de mouvement, aux un ou plusieurs capteurs optiques et aux un ou plusieurs émetteurs optiques ; et
• un dispositif guide-opérateur (150) ;
dans lequel le processeur (110) est configuré pour déterminer si un état de port du dispositif électronique (100) est normal ou anormal, dans lequel, pour déterminer si l'état de port du dispositif électronique (100) est normal ou anormal, le processeur (110) est configuré pour :
o dériver, pour la lumière détectée de chaque couleur parmi les deux couleurs, une pluralité de valeurs de caractéristique, dans lequel chaque valeur de caractéristique parmi la pluralité de valeurs de caractéristique est d'une caractéristique différente parmi une pluralité de caractéristiques différentes ;
o déterminer un état de mouvement actuel d'un utilisateur sur la base des données de mouvement,
o déterminer, pour la lumière de chaque couleur parmi les deux couleurs, un poids pour chaque caractéristique parmi la pluralité de caractéristiques différentes correspondant à l'état de mouvement actuel, dans lequel une exigence plus élevée d'un même état de mouvement sur la stabilité de caractéristique indique un poids plus élevé ;
∘ multiplier, pour la lumière de chaque couleur parmi les deux couleurs, chaque valeur de caractéristique parmi la pluralité de valeurs de caractéristique par son poids correspondant,
o déterminer, pour chaque caractéristique parmi la pluralité de caractéristiques différentes, une différence entre les valeurs de caractéristique correspondantes des lumières des couleurs différentes parmi les deux couleurs qui ont été multipliées par leurs poids correspondants ;
o déterminer une proportion desdites caractéristiques dont la différence est normale parmi la pluralité de caractéristiques différentes, dans lequel chaque caractéristique correspond à une plage de seuil prédéfinie, et une caractéristique dont la différence se situe dans la plage de seuil prédéfinie correspondante est une caractéristique normale ; et
∘ lorsque la proportion desdites caractéristiques dont la différence est normale parmi la pluralité de caractéristiques différentes est supérieure à un premier seuil prédéfini, déterminer, comme état de port du dispositif électronique (100), que le dispositif électronique (100) est porté normalement, sinon, déterminer, comme état de port du dispositif électronique (100), que le dispositif électronique (100) est porté anormalement,
• dans lequel :
o lorsqu'il est déterminé que le dispositif électronique (100) est porté normalement, le dispositif électronique (100) est configuré pour détecter la fréquence cardiaque de l'utilisateur, et
o lorsqu'il est déterminé que le dispositif électronique est porté anormalement, le dispositif guide-opérateur (150) est configuré pour guider l'utilisateur.

2. Dispositif électronique (100) selon la revendication 1, dans lequel il existe au moins deux chemins optiques prédéfinis, chaque chemin optique comprend un émetteur optique (141) et un capteur optique (142), et chaque émetteur optique (141) envoie des lumières d'une ou plusieurs couleurs sur le chemin optique prédéfini.

3. Dispositif électronique (100) selon la revendication 1 ou 2, dans lequel les un ou plusieurs émetteurs optiques (141) et les un ou plusieurs capteurs optiques (142) multiplexent un émetteur optique et un capteur optique dans un module PPG.

4. Dispositif électronique (100) selon l'une quelconque des revendications 1 à 3, dans lequel le processeur (110) est configuré pour s'appuyer en plus, pour déterminer si l'état de port du dispositif électronique (100) est normal, sur une relation entre un second seuil prédéfini et une différence de valeurs de caractéristique d'une même caractéristique qui sont d'une même couleur dans les lumières détectées des deux couleurs et qui se trouvent sur des chemins optiques prédéfinis différents.

5. Dispositif électronique (100) selon l'une quelconque des revendications 1 à 4, dans lequel la différence entre les valeurs de caractéristique est l'une quelconque des suivantes :
une différence de valeur absolue entre les signaux optiques de lumières de couleurs différentes, une différence d'amplitude entre les signaux optiques de lumières de couleurs différentes,
une différence de fréquence de changement entre les signaux optiques de lumières de couleurs différentes, une différence quadratique moyenne entre les signaux optiques de lumières de couleurs différentes et une différence de composante spectrale entre les signaux optiques de lumières de couleurs différentes.

6. Procédé de détection d'une fréquence cardiaque réalisé par un dispositif électronique (100), dans lequel le dispositif électronique (100) comprend :
• un ou plusieurs émetteurs optiques (141) ;
• un ou plusieurs capteurs optiques (142) ;
• un capteur de mouvement configuré pour détecter les données de mouvement ;
• un processeur (110) connecté au capteur de mouvement, aux un ou plusieurs capteurs optiques et aux un ou plusieurs émetteurs optiques ; et
• un dispositif guide-opérateur (150) ;
dans lequel le procédé comprend les étapes suivantes :
∘ l'envoi (S1010), par les un ou plusieurs émetteurs optiques (141), de lumières de deux couleurs sur un chemin optique prédéfini ;
∘ la détection (S1010), par les un ou plusieurs capteurs optiques (142), des lumières sur le chemin optique prédéfini ;
o le fait de déterminer, par le processeur (110), si un état de port du dispositif électronique (100) est normal ou anormal, dans lequel le fait de déterminer si l'état de port du dispositif électronique (100) est normal ou anormal comprend :
- la dérivation, pour la lumière détectée de chaque couleur parmi les deux couleurs, d'une pluralité de valeurs de caractéristique, dans lequel chaque valeur de caractéristique parmi la pluralité de valeurs de caractéristique est d'une caractéristique différente parmi une pluralité de caractéristiques différentes ;
- la détermination d'un état de mouvement actuel d'un utilisateur sur la base des données de mouvement,
- la détermination, pour la lumière de chaque couleur parmi les deux couleurs, d'un poids pour chaque caractéristique parmi la pluralité de caractéristiques différentes correspondant à l'état de mouvement actuel, dans lequel une exigence plus élevée d'un même état de mouvement sur la stabilité de caractéristique indique un poids plus élevé ;
- la multiplication, pour la lumière de chaque couleur parmi les deux couleurs, de chaque valeur de caractéristique parmi la pluralité de valeurs de caractéristique par son poids correspondant,
- la détermination, pour chaque caractéristique parmi la pluralité de caractéristiques différentes, d'une différence entre les valeurs de caractéristique correspondantes des lumières des couleurs différentes parmi les deux couleurs qui ont été multipliées par leurs poids correspondants ;
- la détermination d'une proportion desdites caractéristiques dont la différence est normale parmi la pluralité de caractéristiques différentes, dans lequel chaque caractéristique correspond à une plage de seuil prédéfinie, et une caractéristique dont la différence se situe dans la plage de seuil prédéfinie correspondante est une caractéristique normale ; et
- lorsque la proportion desdites caractéristiques dont la différence est normale parmi la pluralité de caractéristiques différentes est supérieure à un premier seuil prédéfini, le fait de déterminer, comme état de port du dispositif électronique (100), que le dispositif électronique (100) est porté normalement, sinon, le fait de déterminer, comme état de port du dispositif électronique (100), que le dispositif électronique (100) est porté anormalement,
• dans lequel :
o lorsqu'il est déterminé que le dispositif électronique (100) est porté normalement, la détection (S1030), par le dispositif électronique (100), de la fréquence cardiaque de l'utilisateur, et
o lorsqu'il est déterminé que le dispositif électronique est porté anormalement, le guidage, par le dispositif guide-opérateur (150), de l'utilisateur.

7. Procédé selon la revendication 6, dans lequel il existe au moins deux chemins optiques prédéfinis, et chaque chemin optique prédéfini comprend des lumières d'une ou plusieurs couleurs.

8. Procédé selon la revendication 6 ou 7, dans lequel les lumières de deux couleurs sont des signaux PPG.

9. Procédé selon l'une quelconque des revendications 6 à 8, dans lequel le fait de déterminer si l'état de port du dispositif électronique (100) est normal s'appuie en plus sur une relation entre un second seuil prédéfini et une différence de valeurs de caractéristique d'une même caractéristique qui est d'une même couleur dans les lumières détectées de deux couleurs et qui se trouvent sur des chemins optiques prédéfinis différents.

10. Procédé selon l'une quelconque des revendications 6 à 9, dans lequel la différence entre les valeurs de caractéristique de la même caractéristique est l'une quelconque des suivantes : une différence de valeur absolue entre les signaux optiques, une différence d'amplitude entre les signaux optiques, une différence de fréquence de changement entre les signaux optiques, une différence quadratique moyenne entre les signaux optiques et une différence de composante spectrale entre les signaux optiques.

11. Produit de programme informatique comprenant des instructions lisibles par ordinateur, dans lequel, lorsqu'un ordinateur lit et exécute les instructions lisibles par ordinateur, l'ordinateur est amené à réaliser le procédé selon l'une quelconque des revendications 6 à 10.
